# EUROPEAN PATENT APPLICATION

(11) **EP 4 545 188 A1**
(43) Date of publication of application: **30.04.2025**
(21) Application number: 23867455.0
(22) Date of filing: 18.09.2023
(51) Int. Cl.: B05B 17/06, G01H 13/00

(54) **ULTRASONIC ATOMIZER, AND RESONANT-FREQUENCY DETERMINATION METHOD BASED ON ULTRASONIC ATOMIZER**

(30) Priority: 22.09.2022 CN 202211159798
(71) Applicant: Shenzhen First Union Technology Co., Ltd., Shenzhen, Guangdong 518000 (CN)
(72) Inventor: LI, Xinjun, Shenzhen, Guangdong 518000 (CN); XU, Zhongli, Shenzhen, Guangdong 518000 (CN); LI, Yonghai, Shenzhen, Guangdong 518000 (CN)
(74) Representative: Ran, Handong
(86) International application number: PCT/CN2023/119425
(87) International publication number: WO 2024/061166

(57) **Abstract**

An ultrasonic atomizer (100), and a resonant-frequency determination method based on the ultrasonic atomizer (100). The ultrasonic atomizer (100) comprises a storage cavity (11), an ultrasonic atomization piece (12), a control circuit (13) and a power source (14). The storage cavity (11) stores an atomization matrix. The ultrasonic atomization piece (12) generates oscillation to atomize the atomization matrix. The control circuit (13) comprises a controller (131), a driving branch (132) and an impedance branch (133), wherein the driving branch (132) is connected to the power source (14) and the controller (131), and the driving branch (132) generates a driving voltage in response to a first pulse signal; the impedance branch (133) is connected between the driving branch (132) and the ultrasonic atomization piece (12), the impedance branch (133) makes a combined impedance of the impedance branch (133) and the ultrasonic atomization piece (12) match the impedance of the driving branch (132), and the impedance branch (133) comprises a first capacitor (C1), which is connected to the ultrasonic atomization piece (12) in parallel; and the controller (131) acquires a first voltage of the ultrasonic atomization piece (12), and determines a resonant frequency of the ultrasonic atomization piece (12) according to the first voltage.

## Description

### CROSS-REFERENCE TO RELATED APPLICATIONS

This application claims priority to Chinese Patent Application No. 202211159798.5, filed with China National Intellectual Property Administration on September 22, 2022 and entitled "ULTRASONIC ATOMIZER, AND RESONANT-FREQUENCY DETERMINATION METHOD BASED ON ULTRASONIC ATOMIZER", which is incorporated herein by reference in its entirety.

### TECHNICAL FIELD

This application relates to the technical field of electronic circuits, and in particular, to an ultrasonic atomizer, and a resonant-frequency determination method based on an ultrasonic atomizer.

### BACKGROUND

In daily life, ultrasonic atomizers may be used in a plurality of fields such as humidifying, perfuming, sterilizing, decorating, medical atomization, and e-cigarettes. Resonant frequency is a crucial parameter for the ultrasonic atomizers. However, due to changes of various factors such as temperature, environment, aging of components, and an externally applied force, the resonant frequency of an ultrasonic atomization piece in the ultrasonic atomizer may shift. The shift of the resonant frequency may reduce work efficiency of the ultrasonic atomization piece, and even damage the ultrasonic atomization piece. Therefore, to enable a system to have relatively large output power, the ultrasonic atomization piece usually needs to be operated at the resonant frequency.

In a related technology, to determine a resonant frequency of an ultrasonic atomization piece, a phase detection means is usually used, that is, a voltage and a current of the ultrasonic atomization piece are collected, a phase relationship between the voltage and the current is compared, and a driving frequency is constantly adjusted, so that a phase difference between the voltage and the current is 0, thereby determining the resonant frequency of the ultrasonic atomization piece.

However, costs of the foregoing mode are relatively high, circuits are relatively complex, and implementation difficulty is relatively high. This is not beneficial to saving costs and achieving miniaturization, and has poor practicability.

### SUMMARY

This application aims to provide an ultrasonic atomizer and a frequency determination method based on an ultrasonic atomizer, which can determine a resonant frequency of an ultrasonic atomization piece in a simpler mode, which is beneficial to reducing costs and achieve miniaturization, and has strong practicality.

To achieve the foregoing purpose, according to a first aspect, this application provides an ultrasonic atomizer, including:
a storage cavity, configured to store an atomization matrix;
an ultrasonic atomization piece, configured to generate oscillation to atomize the atomization matrix;
a control circuit and a power supply, where
the control circuit includes:
   a controller and a driving branch, where the driving branch is connected to each of the power supply and the controller, the driving branch is configured to generate a driving voltage in response to a first pulse signal output by the controller, and the driving voltage is used for driving the ultrasonic atomization piece;
   an impedance branch, connected between the driving branch and the ultrasonic atomization piece, where the impedance branch is configured to match a combined impedance of the impedance branch and the ultrasonic atomization piece with an impedance of the driving branch, and the impedance branch includes a first capacitor connected in parallel to the ultrasonic atomization piece; and
   the controller is configured to obtain a first voltage of the ultrasonic atomization piece, and determine a resonant frequency of the ultrasonic atomization piece according to the first voltage.

In an optional mode, the controller is specifically configured to:
obtain the first voltage after a first time, and determine the resonant frequency of the ultrasonic atomization piece according to the first voltage, where the first time is later than a time at which the ultrasonic atomization piece is started.

In an optional mode, duration between the time at which the ultrasonic atomization piece is started and the first time is any duration of [10 µs, 100 µs].

In an optional mode, the control circuit further includes a first conversion branch;
the first conversion branch is connected between the ultrasonic atomization piece and the controller, and the first conversion branch is configured to convert a waveform of a first voltage into a first waveform fluctuating within a preset range; and
the controller is further configured to determine the resonant frequency of the ultrasonic atomization piece according to a second voltage corresponding to the first waveform.

In an optional mode, the first conversion branch includes an energy storage sub-branch; and
the energy storage sub-branch is connected between the ultrasonic atomization piece and the controller, and the energy storage sub-branch is configured to store energy in response to the first voltage; and
if a maximum change value of the voltage obtained by the energy storage of the energy storage sub-branch is less than a preset change threshold within first duration, a waveform of the voltage obtained by energy storage of the energy storage sub-branch is taken as the first waveform.

In an optional mode, the controller is specifically configured to:
output N driving frequencies to drive the ultrasonic atomization piece, where N is an integer ≥2;
obtain first waveforms under an action of M driving frequencies of the N driving frequencies to obtain M first waveforms, where 2≤M≤N;
obtain the second voltage corresponding to the first waveform; and
determine the resonant frequency of the ultrasonic atomization piece according to a frequency corresponding to a minimum value of the second voltage.

In an optional mode, the control circuit further includes a voltage amplification branch; and
the voltage amplification branch is connected between the energy storage sub-branch and the controller, and the voltage amplification branch is configured to: amplify a voltage obtained by the energy storage of the energy storage sub-branch, and output a second waveform to the controller; and
the controller is further configured to determine the resonant frequency of the ultrasonic atomization piece according to a third voltage corresponding to the second waveform.

In an optional mode, the control circuit further includes a second conversion branch;
the second conversion branch is connected between the ultrasonic atomization piece and the controller, and the second conversion branch is configured to: convert a waveform of the first voltage into a third waveform having an interval voltage signal, and output the third waveform to the controller; and
the controller is further configured to determine the resonant frequency of the ultrasonic atomization piece according to a fourth voltage in the third waveform corresponding to the voltage signal.

In an optional mode, the second conversion branch includes a peak value obtaining sub-branch; and
the peak value obtaining sub-branch is connected between the ultrasonic atomization piece and the controller, and the peak value obtaining sub-branch is configured to obtain a peak value of the waveform of the first voltage to output the third waveform, where the third waveform generates the voltage signal once each time the peak value is obtained.

In an optional mode, the controller is specifically configured to:
output N driving frequencies to drive the ultrasonic atomization piece, where N is an integer ≥2;
obtain a third waveform under an action of M driving frequencies of the N driving frequencies to obtain M third waveforms, 2≤M≤N;
obtain the fourth voltage corresponding to the third waveform; and
determine the resonant frequency of the ultrasonic atomization piece according to a frequency corresponding to a minimum value of the fourth voltage.

According to a second aspect, this application provides an ultrasonic atomizer, including:
a storage cavity, configured to store an atomization matrix;
an ultrasonic atomization piece, configured to generate oscillation to atomize the atomization matrix;
a control circuit and a power supply, where
the control circuit includes a controller, a driving branch, and an impedance branch;
the driving branch includes a power supply sub-branch, a switch sub-branch, a capacitive sub-branch, and a resonant sub-branch, where the power supply sub-branch is connected to the power supply, the power supply sub-branch is configured to generate direct current according to the power supply, the switch sub-branch is connected to each of the controller and the power supply sub-branch, the switch sub-branch is configured to be turned on or turned off in response to a first pulse signal output by the controller to generate a pulse voltage according to the direct current, the capacitive sub-branch is connected to the switch sub-branch, and the capacitive sub-branch is configured to implement soft turn-on or soft turn-off of the switch sub-branch, and the resonant sub-branch is connected to each of the power supply sub-branch, the switch sub-branch, and the impedance branch, and the resonant sub-branch is configured to resonate in response to turn-on and turn-off of the switch sub-branch to output a driving voltage according to the pulse voltage to drive the ultrasonic atomization piece;
the impedance branch is connected between the driving branch and the ultrasonic atomization piece, and the impedance branch is configured to match a combined impedance of the impedance branch and the ultrasonic atomization piece with an impedance of the driving branch; and
the controller is configured to obtain a voltage of the ultrasonic atomization piece, and determine a resonant frequency of the ultrasonic atomization piece according to the voltage of the ultrasonic atomization piece.

In an optional mode, the controller is specifically configured to:
output N driving frequencies to drive the ultrasonic atomization piece, where N is an integer ≥2;
obtain detection signals corresponding to voltages of the ultrasonic atomization piece under an action of M driving frequencies of the N driving frequencies to obtain M detection signals, where 2≤M≤N; and
determine the resonant frequency of the ultrasonic atomization piece according to a frequency corresponding to a minimum value of the M detection signals.

According to a third aspect, this application provides a resonant frequency determination method based on an ultrasonic atomizer, including:
outputting N driving frequencies to drive an ultrasonic atomization piece in the ultrasonic atomizer, where N is an integer ≥2;
obtaining detection signals corresponding to voltages of the ultrasonic atomization piece under an action of M driving frequencies of the N driving frequencies to obtain M detection signals; and
determining a resonant frequency of the ultrasonic atomization piece according to a frequency corresponding to a minimum value of the voltage corresponding to the M detection signals.

In an optional mode, the obtaining detection signals corresponding to voltages of the ultrasonic atomization piece under an action of M driving frequencies of the N driving frequencies includes:
performing a charge accumulation operation according to a voltage of the ultrasonic atomization piece; and
taking, if a maximum change value of the voltage obtained by the charge accumulation within first duration is less than a preset change threshold, a voltage waveform obtained by charge accumulation within the first duration as a detection signal.

In an optional mode, the obtaining detection signals corresponding to voltages of the ultrasonic atomization piece under an action of M driving frequencies of the N driving frequencies includes:
performing a charge accumulation operation according to a voltage of the ultrasonic atomization piece until a voltage obtained by charge accumulation is equal to a maximum value of the voltage of the ultrasonic atomization piece, and recording the voltage obtained by the charge accumulation as a reference voltage; and
outputting a voltage signal when the voltage of the ultrasonic atomization piece is equal to the reference voltage, where the voltage signal is a detection signal.

An ultrasonic atomizer provided in this application includes a storage cavity, an ultrasonic atomization piece, a control circuit, and a power supply. The control circuit includes a controller, a driving branch, and an impedance branch. The driving branch is connected to each of the power supply and the controller, and the impedance branch is connected between the driving branch and the ultrasonic atomization piece. The driving branch is configured to generate a driving voltage in response to a first pulse signal output by the controller, and the driving voltage is used for driving the ultrasonic atomization piece. The impedance branch is configured to match a combined impedance of the impedance branch and the ultrasonic atomization piece with an impedance of the driving branch, where the impedance branch includes a first capacitor connected in parallel to the ultrasonic atomization piece. The controller is configured to obtain a first voltage of the ultrasonic atomization piece, and determine a resonant frequency of the ultrasonic atomization piece according to the first voltage. In the foregoing modes, the resonant frequency of the ultrasonic atomization piece can be determined by obtaining the voltage of the ultrasonic atomization piece through a simple circuit, which is beneficial to reducing costs and achieving miniaturization, and has strong practicality.

### BRIEF DESCRIPTION OF THE DRAWINGS

One or more embodiments are exemplarily described with reference to corresponding accompanying drawings, and the exemplary descriptions do not construe a limitation to the embodiments. Components having same reference numerals in the accompanying drawings represent similar components, and unless otherwise particularly stated, figures in the accompanying drawings are not drawn to scale.
FIG. 1 is a schematic structural diagram of an ultrasonic atomizer according to an embodiment of this application;
FIG. 2 is a schematic structural diagram of an ultrasonic atomizer according to another embodiment of this application;
FIG. 3 is a schematic structural diagram of a control circuit according to an embodiment of this application;
FIG. 4 is a schematic structural diagram of a circuit structure according to another embodiment of this application;
FIG. 5 is a schematic structural diagram of a circuit of a first conversion branch and a voltage amplification branch according to an embodiment of this application;
FIG. 6 shows waveform graphs of first voltages and first waveforms under different driving frequencies according to an embodiment of this application;
FIG. 7 is a schematic structural diagram of a control circuit according to an embodiment of this application;
FIG. 8 is a schematic structural diagram of a circuit of a second conversion branch according to an embodiment of this application;
FIG. 9 is a schematic structural diagram of a control circuit according to another embodiment of this application;
FIG. 10 is a schematic structural diagram of a circuit of a driving branch and a circuit of an impedance branch according to an embodiment of this application;
FIG. 11 is a flowchart of a resonant-frequency determination method based on an ultrasonic atomizer according to an embodiment of this application;
FIG. 12 is a schematic diagram of an implementation of step 1102 shown in FIG. 11 according to an embodiment of this application; and
FIG. 13 is a schematic diagram of another implementation of step 1102 shown in FIG. 11 according to an embodiment of this application.

### DETAILED DESCRIPTION

To make the objectives, technical solutions, and advantages of embodiments of this application clearer, the following clearly and completely describes the technical solutions in embodiments of this application with reference to accompanying drawings in embodiments of this application. It is clear that the described embodiments are merely some rather than all of embodiments of this application. All other embodiments obtained by a person of ordinary skill in the art based on the embodiments of this application without making creative efforts shall fall within the protection scope of this application.

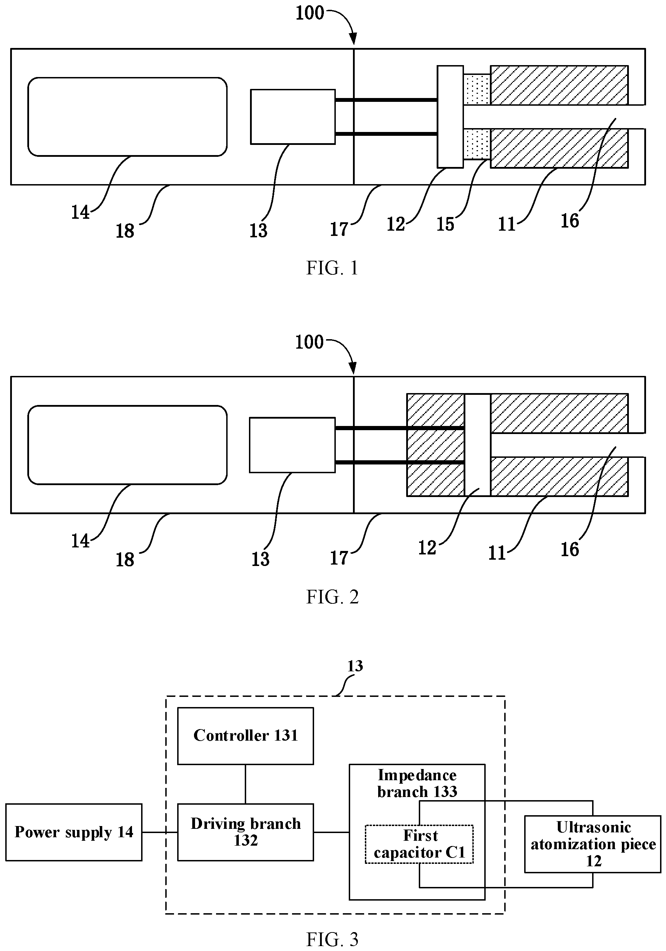

Refer to FIG. 1, which is a schematic structural diagram of an ultrasonic atomizer according to an embodiment of this application. As shown in FIG. 1, the ultrasonic atomizer 100 includes a storage cavity 11, an ultrasonic atomization piece 12, a control circuit 13, and a power supply 14.

The storage cavity 11 is configured to store an atomization matrix. The atomization matrix may include different substances according to different use scenarios. For example, in a field of e-cigarette atomization, nicotine and/or a flavoring agent and/or an aerosol generated substance (for example, glycerol) may be included; and for another example, in a field of medical atomization, solutions such as medicines and/or normal saline that are beneficial to disease treatment or health may be included.

The ultrasonic atomization piece 12 is in fluid communication with the storage cavity 11, which may be that the ultrasonic atomization piece 12 is directly arranged in the storage cavity 11, or an atomization cavity in which the ultrasonic atomization piece 12 is located is in direct communication with the storage cavity 11, or the ultrasonic atomization piece 12 is in fluid communication with the storage cavity 11 through a medium. The ultrasonic atomization piece 12 is configured to generate oscillation to atomize the atomization matrix, that is, the atomization matrix transferred onto or near the ultrasonic atomization piece 12 is atomized into an aerosol through vibration. Specifically, during use, the ultrasonic atomization piece 12 disperses the atomization matrix through high-frequency vibration (preferably, a vibration frequency is in a range of 1.7 MHz to 4.0 MHz, which exceeds a human hearing range and belongs to an ultrasonic frequency band) to generate an aerosol with naturally suspended particles.

The control circuit 13 is electrically connected to the ultrasonic atomization piece 12, and the control circuit 13 is configured to provide a driving voltage and a driving current for the ultrasonic atomization piece 12 according to the power supply 14. In an implementation, the control circuit 13 may be arranged on a printed circuit board (PCB).

The power supply 14 is configured to supply power. In an implementation, the power supply 14 is a battery. The battery may be a lithium-ion battery, a lithium metal battery, a lead-acid battery, a nickelcadmium battery, a nickel-metal hydride battery, a lithium-sulfur battery, a lithium-air battery, a sodium-ion battery, or the like. This is not limited herein. In terms of scale, the battery in the embodiments of this application may be a battery cell, or may be a battery module including a plurality of battery cells connected in series and/or connected in parallel. This is not limited herein. Certainly, in other embodiments, the battery may alternatively include more or fewer components, or have different component configurations. This is not limited in the embodiments of this application.

In an embodiment, the ultrasonic atomizer 100 further includes a liquid transfer medium 15 and an air outlet channel 16. The liquid transfer component 15 is configured to transfer a liquid atomization matrix between the storage cavity 11 and the ultrasonic atomization piece 12. The air outlet channel 16 is configured to output inhalable vapor or an aerosol generated by the atomization matrix for a user to inhale.

The ultrasonic atomizer 100 may be integrated or assembled. In an implementation, when the ultrasonic atomizer 100 is assembled, the ultrasonic atomizer 100 further includes a power supply mechanism and an ultrasonic atomizer body. The ultrasonic atomizer body includes a first housing 17, and the power supply mechanism includes a second housing 18.

In an embodiment, the first housing 17 is detachably connected to the second housing 18. For example, the first housing 17 may be detachably connected to the second housing 18 through a buckle structure, a magnetic structure, or the like. The first housing 17 and the second housing 18 jointly play a role in accommodating and protecting other components. The storage cavity 11, the ultrasonic atomization piece 12, the liquid transfer component 15, and the air outlet channel 16 are all provided in the first housing 17, and the control circuit 13 and the power supply 14 are both arranged in the second housing 18.

The first housing 17 is detachably aligned with the second housing 18 in a functional relationship. The second housing 18 may be connected the first housing 17 by using various mechanisms to generate threaded engagement, press-fit engagement, interference fit, magnetic engagement, or the like. In some implementations, when the first housing 17 and the second housing 18 are assembled and configured, the ultrasonic atomizer 100 may be basically in a shape of a clavate, a flat cylinder, a rod, a column, or the like.

The first housing 17 and the second housing 18 may be formed of any suitable material that is structurally sound. In some examples, the first housing 17 and the second housing 18 may be formed by a metal or an alloy such as stainless steel or aluminum. Other appropriate materials include various plastics (for example, polycarbonate), metal-plating over plastic, ceramics, and the like.

It is to be noted that, a hardware structure of the ultrasonic atomizer 100 shown in FIG. 1 is only an example. Moreover, the ultrasonic atomizer 100 may have more or fewer components than those shown in the figure, or may combine two or more components, or may have a different component configuration. Various components shown in the figure may be implemented in hardware including one or more signal processing circuits and/or application-specific integrated circuits, software, or a combination of hardware and software. For example, as shown in FIG. 2, the ultrasonic atomization piece 12 may be arranged in the storage cavity 11, so as to simplify a structure.

In addition, it may be understood that, the ultrasonic atomizer 100 shown in FIG. 1 or FIG. 2 may be applied to a plurality of different scenarios and play different roles. This is not specifically limited in this embodiment of this application. For example, in an embodiment, the ultrasonic atomizer 100 is applied to a medical field. In this case, the ultrasonic atomizer 100 may be a medical atomizer. The medical atomizer can achieve an auxiliary treatment effect by atomizing a medical liquid added inside the medical atomizer and enabling a patient to inhale. For another example, in another embodiment, the ultrasonic atomizer 100 may alternatively serve as an electronic product, for example, an e-cigarette. The e-cigarette is an electronic product that turns, by means of atomizing and the like, a nicotine solution and the like into aerial fog for a user to inhale.

Refer to FIG. 3, which is a schematic structural diagram of a connection between a control circuit 13 and a power supply 14. As shown in FIG. 3, the control circuit 13 includes a controller 131, a driving branch 132, and an impedance branch 133. The driving branch 132 is connected to each of the power supply 14 and the controller 131. The impedance branch 133 is connected between the driving branch 132 and the ultrasonic atomization piece 12, and the impedance branch 133 includes a first capacitor C1 connected in parallel to the ultrasonic atomization piece 12.

Specifically, the driving branch 132 is configured to generate a driving voltage in response to a first pulse signal output by the controller 131, and the driving voltage is used for driving the ultrasonic atomization piece 12. The impedance branch 133 is configured to match a combined impedance of the impedance branch 133 and the ultrasonic atomization piece 12 with an impedance of the driving branch 132, so as to reduce a reactive power part of a combination of the impedance branch 133 and the ultrasonic atomization piece 12. The controller 131 is configured to obtain a first voltage of the ultrasonic atomization piece 12, and determine a resonant frequency of the ultrasonic atomization piece 12 according to the first voltage.

In a related technology, the resonant frequency of the ultrasonic atomization piece 12 is usually obtained in two modes. A first mode is a mode of determining the resonant frequency by collecting an output current of the power supply 14. Specifically, because the ultrasonic atomization piece 12 has a minimum impedance and the power supply 14 has a maximum output current when operating at the resonant frequency, the ultrasonic atomization piece may be driven by a plurality of frequencies, an output current corresponding to each frequency is collected, and a frequency corresponding to a maximum value of the collected output current is the resonant frequency.

However, for this application, because there is the first capacitor C1 connected in parallel to the ultrasonic atomization piece, a branch in which the first capacitor C1 is located will generate a shunt current of the current, and then a maximum value of the collected output current does not correspond to the resonant frequency any longer, that is, the frequency corresponding to the maximum value of the output current is not the resonant frequency. Therefore, the mode of determining the resonant frequency by collecting the output current of the power supply 14 is not applicable to the ultrasonic atomizer 100 in this embodiment of this application.

A second mode is to use a phase detection means, that is, a voltage and a current of the ultrasonic atomization piece 12 are collected, a phase relationship between the voltage and the current is compared, and a driving frequency is constantly adjusted, so that a phase difference between the voltage and the current is 0, thereby determining the resonant frequency of the ultrasonic atomization piece 12. However, costs of this mode are relatively high, circuits are relatively complex, and implementation difficulty is relatively high. This is not beneficial to saving costs and achieving miniaturization, and has poor practicability.

However, for this application, the resonant frequency of the ultrasonic atomization piece 12 by using a simpler circuit. Specifically, when the voltage output by the power supply 14 is kept unchanged, if the ultrasonic atomization piece 12 operates at a resonant frequency point, and the impedance of the ultrasonic atomization piece 12 is minimum, in this case, a voltage at both ends of the ultrasonic atomization piece 12 is minimum. In addition, because the first capacitor C1 is connected in parallel to the ultrasonic atomization piece 12, the first capacitor C1 does not affect the voltage at the both ends of the ultrasonic atomization piece 12. In conclusion, the resonant frequency of the ultrasonic atomization piece 12 can be determined by obtaining a first voltage of the ultrasonic atomization piece 12. Moreover, the used circuit structure is relatively simple, which is beneficial to reducing costs and achieving miniaturization, and has strong practicability.

In an implementation, the controller 131 is further specifically configured to: obtain a first voltage after a first time, and determine a resonant frequency of the ultrasonic atomization piece according to the first voltage. The first time is later than a time at which the ultrasonic atomization piece is started.

After the ultrasonic atomization piece 12 is started, the voltage at both ends of the ultrasonic atomization piece 12 gradually decreases from a maximum voltage to a stable state. When the ultrasonic atomization piece 12 is in the stable state, the voltage at the both ends of the ultrasonic atomization piece 12 fluctuates up and down within a range, so that the first voltage is obtained only when the ultrasonic atomization piece 12 is in the stable state, and the resonant frequency of the ultrasonic atomization piece 12 can be further determined. Duration between the time at which the ultrasonic atomization piece 12 is started and the first time, that is, duration in which the voltage at the both ends of the ultrasonic atomization piece 12 decreases from the maximum voltage to the stable state may be set according to an actual application case. This is not specifically limited in this implementation of this application. For example, in an embodiment, duration between the time at which the ultrasonic atomization piece 12 is started and the first time is any duration of [10 µs, 100 µs]. Moreover, to maintain stability and accuracy of a detection result, the duration may be set to a relatively large value, for example, 100 µs.

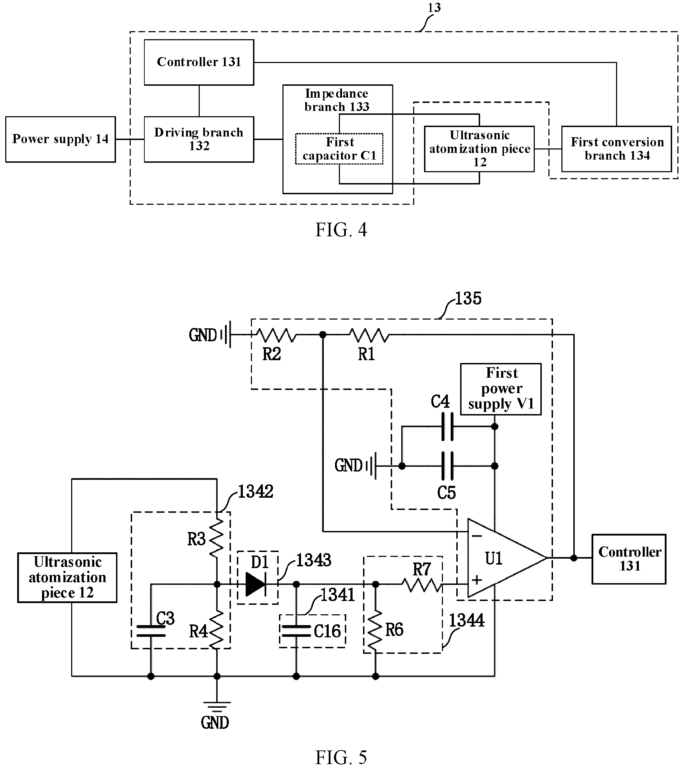

In an embodiment, as shown in FIG. 4, the control circuit 13 further includes a first conversion branch 134. The first conversion branch 134 is connected between the ultrasonic atomization piece 12 and the controller 131.

The first conversion branch 134 is configured to convert a waveform of a first voltage into a first waveform fluctuating within a preset range. The controller 131 is further configured to determine the resonant frequency of the ultrasonic atomization piece 12 according to a second voltage corresponding to the first waveform. The preset range may be set according to an actual application case. This is not specifically limited in this embodiment of this application.

In this embodiment, the waveform of the first voltage is a waveform that changes rapidly. If the waveform needs to be directly sampled, the controller 131 with a relatively high sampling speed and precision is needed, and such a controller has relatively high costs. After the waveform of the first voltage is converted into the first waveform fluctuating within the preset range, requirements on the sampling speed and precision can be greatly reduced, so that the controller 131 with a relatively low sampling speed and precision can be selected to use, thereby not only implementing sampling of the first waveform, but also reducing costs.

Referring to FIG. 4 and FIG. 5 together, in an embodiment, the first conversion branch includes an energy storage sub-branch 1341. The energy storage sub-branch 1341 is connected between the ultrasonic atomization piece 12 and the controller 131.

The energy storage sub-branch 1341 is configured to store energy in response to the first voltage. If a maximum change value of the voltage obtained by the energy storage of the energy storage sub-branch 1341 is less than a preset change threshold within first duration, a waveform of the voltage obtained by energy storage of the energy storage sub-branch 1341 is taken as the first waveform.

Driven by the first voltage, charge on the energy storage sub-branch 1341 constantly accumulates. A waveform of the voltage obtained by charge accumulation in this case is taken as the first waveform when a maximum change value (that is, a difference between a maximum value and a minimum value within first duration) of the voltage obtained by the charge accumulation is maintained less than a preset change threshold within the first duration. In this case, it may be considered that the charge is maintained in a stable state, that is, the first waveform is a stable signal.

The preset change threshold may be set according to an actual application case. This is not specifically limited in this embodiment of this application. For example, in an embodiment, the preset change threshold may be set to 0.2 v. In this case, if the voltage obtained by the charge accumulation is maintained in a range of [1v, 1.1 v], and maintaining duration is greater than or equal to the first duration, the maximum change value of the voltage obtained by the charge accumulation is 1.1-1=0.1 v, which is less than 0.2 v. In this case, it may be considered that the voltage obtained by the charge accumulation is a stable voltage, and the first waveform may be obtained.

The first duration may be set according to an actual application case. This is not specifically limited in this embodiment of this application. For example, in an implementation, the first duration is set to any duration in (0, 10 ms], for example, 10 ms. If the maximum change value of the voltage obtained by the charge accumulation can be maintained less than the preset change threshold within 10 ms, a fluctuation amplitude of the voltage obtained by the charge accumulation within 10 ms is relatively small. In this case, it may be considered that the first waveform obtained by the charge accumulation is a stable waveform, and a second voltage corresponding to the first waveform is a stable voltage. By setting the first duration, the first waveform can be more accurately determined as a relatively stable waveform, thereby reducing probability of misjudgment.

For another example, in another implementation, the first duration is set to duration greater than or equal to five sampling periods, and each sampling period is any duration in (0, 100 µ s]. The sampling period is a period in which the voltage obtained by the charge accumulation is sampled each time. If the voltage obtained by the charge accumulation is constantly sampled for more than five times, and maximum change values of the sampled voltages obtained by the charge accumulation are all less than the preset change threshold, it may be determined that the first waveform is a relatively stable waveform.

For example, in an implementation, the preset change threshold is set to 0.2 v, the first duration is set to duration equal to five sampling periods, a voltage obtained by sampling for the first time is 0.4 v, a voltage obtained by sampling for the second time is 0.5 v, a voltage obtained by sampling for the third time is 0.5 v, a voltage obtained by sampling for the fourth time is 0.4 v, and a voltage obtained by sampling for the fifth time is 0.5 v. In this embodiment, the maximum change value of the voltage is 0.5-0.4=0.1 v<0.2 v. In this case, a current waveform of the voltage obtained by the charge accumulation may be taken as the first waveform, and a second voltage corresponding to the first waveform may be 0.5 v.

In this embodiment, in a mode of sampling for a plurality of times, a sampling error caused by fluctuation of the voltage due to environment interference and the like can be avoided, and similarly, a probability of misjudgment can be reduced. In addition, by setting the sampling period to any duration between (0, 100 µs], a probability that a voltage can be sampled can be improved.

Then, because there is a correspondence between the first voltage and the second voltage corresponding to the first waveform, magnitude of the driving voltage may be determined by obtaining the second voltage.

In addition, in this embodiment, no matter whether the first voltage is a rapidly changing quantity (that is, a signal with a relatively high frequency) or a slowly changing quantity (that is, a signal with a relatively low frequency), a charge accumulation process can be implemented. Therefore, the method may be applicable to signals of different frequencies, that is, may be applicable to different application scenarios, and has strong practicability. In addition, because the voltage obtained by energy storage of the energy storage sub-branch 1341 is a variable obtained after the charge accumulation, the variable has a relatively low requirement on processing of the controller. As the sampling frequency decreases, price of the controller decreases. In this case, a controller with a relatively low sampling frequency may be selected to reduce costs while meeting a sampling requirement for the first waveform.

Further, in an embodiment, the controller 131 is specifically configured to: output N driving frequencies to drive the ultrasonic atomization piece, where N is an integer ≥ 2; obtain first waveforms under an action of M driving frequencies of the N driving frequencies to obtain M first waveforms, where 2≤M≤N; obtain the second voltage corresponding to the first waveform; and determine a resonant frequency of the ultrasonic atomization piece according to a frequency corresponding to a minimum value of the second voltage.

When the ultrasonic atomization piece 12 operates at the resonant frequency, the first voltage at both ends of the ultrasonic atomization piece 12 is a minimum voltage. Therefore, if a detected first voltage tends to increase as a driving frequency increases, the first voltage does not need to be collected at a subsequent driving frequency, that is, the first waveform does not need to be collected to improve work efficiency. That is, the first waveform may only need to be collected at M driving frequencies of the N driving frequencies, or the first waveform may need to be collected at all driving frequencies of a plurality of driving frequencies.

Then, each first waveform may determine one second voltage, and M first waveforms may determine M second voltages in total. A minimum value of the M second voltages is obtained, and a frequency corresponding to the minimum value is the resonant frequency of the ultrasonic atomization piece 12.

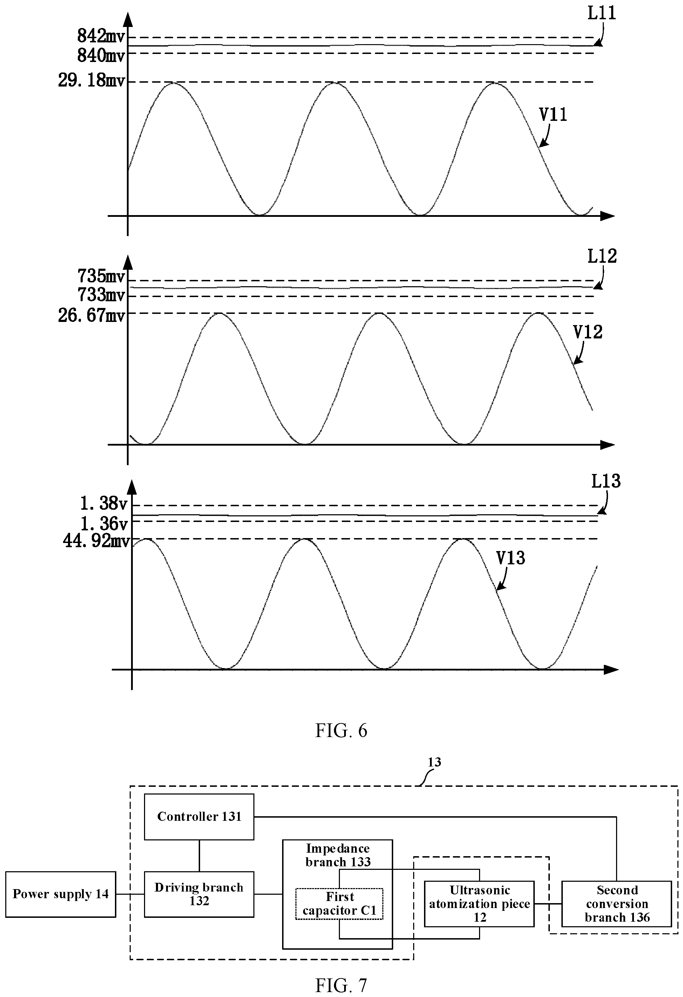

Refer to FIG. 6, which exemplarily shows first voltages and first waveforms obtained by sampling an ultrasonic atomization piece 12 with a resonant frequency of 2.94 MHz under an action of different driving frequencies. A curve V11 indicates a first voltage when a driving frequency is 2.92 MHz; a curve L11 indicates a first waveform when a driving frequency is 2.92 MHz; a curve V12 indicates a first voltage when a driving frequency is 2.94 MHz; a curve L12 indicates a first waveform when a driving frequency is 2.94 MHz; a curve V13 indicates a first voltage when a driving frequency is 2.96 MHz; and a curve L13 indicates a first waveform when a driving frequency is 2.96 MHz.

As shown in FIG. 6, when the driving frequency is 2.92 MHz, a change range of the first waveform is [840 mv, 842 mv], a preset change threshold is 2 mv, and a second voltage corresponding to the first waveform may be 841 mv; when the driving frequency is 2.94 MHz, a change range of the first waveform is [733 mv, 735 mv], and a preset change threshold is 2 mv, and a second voltage corresponding to the first waveform may be 734 mv; and when the driving frequency is 2.96 MHz, a change range of the first waveform is [1.36 v, 1.38 v], and a preset change threshold is 2 mv, and a second voltage corresponding to the first waveform may be 1.37 v. It can be learned that, when the driving frequency is 2.94 MHz and is exactly the resonant frequency, the second voltage corresponding to the first waveform is minimum, and opposite conditions also hold true. Therefore, if a minimum value of the M second voltages at the M driving frequencies is obtained, a frequency corresponding to the minimum value is the resonant frequency of the ultrasonic atomization piece 12.

In an embodiment, referring back to FIG. 5, the first conversion branch 134 further includes a first preprocessing sub-branch 1342, a rectification sub-branch 1343, and a voltage limiting and current limiting sub-branch 1344. The first preprocessing sub-branch 1342 is connected to the ultrasonic atomization piece 12, the rectification sub-branch 1343 is connected to the first preprocessing sub-branch 1342, and the voltage limiting and current limiting sub-branch 1344 is connected to each of the rectification sub-branch 1343, the energy storage sub-branch 1341, and the controller 131.

Specifically, a first end of the first preprocessing sub-branch 1342 is connected to a first end of the ultrasonic atomization piece 12, a second end of the first preprocessing sub-branch 1342 is connected to a first end of the rectification sub-branch 1343, a second end of the rectification sub-branch 1343 is connected to each of a first end of the energy storage sub-branch 1341 and a first end of the voltage limiting and current limiting sub-branch 1344, and a second end of the voltage limiting and current limiting sub-branch 1344 is connected to the controller 131.

The first preprocessing sub-branch 1342 is configured to: divide and filter the first voltage of the ultrasonic atomization piece 12, and output a first sub-voltage. The rectification sub-branch 1343 is configured to rectify the first sub-voltage, so that the energy storage sub-branch 1341 stores energy in response to the first sub-voltage, and outputs a first detection voltage. The voltage limiting and current limiting sub-branch 1344 is configured to output a first waveform fluctuating within a preset range to the controller 131 after performing voltage limiting and current limiting on the first detection voltage, so that the controller 131 determines a resonant frequency of the ultrasonic atomization piece 12 according to a second voltage corresponding to the first waveform.

In this embodiment, in a working process of the ultrasonic atomization piece 12, to determine a value of the first voltage, first, the first preprocessing sub-branch 1342 is configured to divide and filter the first voltage. Through voltage division processing, an amplitude of a first driving signal can be reduced to reduce a voltage input to the controller 131, which is beneficial to protecting the controller 131. Through filtering processing, a possible high-voltage pulse signal is filtered out, so as to protect a subsequent electronic component, for example, the controller 131.

Then, the first preprocessing sub-branch 1342 outputs the first sub-voltage, and the first sub-voltage is rectified by the rectification sub-branch 1343, so as to rectify the first sub-voltage into a signal capable of charging the energy storage sub-branch 1341.

Then, the energy storage sub-branch 1341 stores energy based on the rectified first sub-voltage, and a voltage of the energy storage sub-branch 1341 gradually increases until the voltage of the energy storage sub-branch 1341 is maintained in a relatively stable state. For example, if the voltage of the energy storage sub-branch 1341 is within a preset voltage range, it may be considered that the voltage of the energy storage sub-branch 1341 is maintained in the relatively stable state. In this case, the voltage of the energy storage sub-branch 1341 is recorded as the first detection voltage, and the first detection voltage is transmitted to the voltage limiting and current limiting sub-branch 1344. Even if the first voltage is a rapidly changing quantity (that is, a signal with a relatively high frequency), the energy storage sub-branch 1341 can perform a plurality of accumulations according to the first sub-voltage to obtain a variable that can reflect an amplitude and a frequency of the first sub-voltage, and then input the variable to the controller 131. It can be learned that, a signal received by the controller 131 is not the rapidly changing quantity, but is a variable obtained through accumulation. Therefore, a processing requirement on the controller 131 is not high. In other words, even if the controller 131 with a relatively low sampling frequency is selected, a sampling requirement on the first detection voltage can be met. However, as a sampling frequency decreases, price of the controller 131 also decreases, so that costs can be reduced by selecting the controller 131 with the relatively low sampling frequency.

After receiving the first detection voltage, the voltage limiting and current limiting sub-branch 1344 limits a voltage of the first detection voltage to prevent an excessively large voltage increase of the first detection voltage, and limits the current of the first detection voltage to prevent an excessively high current from flowing into the controller 131, so as to protect the controller 131. Then, the voltage limiting and current limiting sub-branch 1344 further outputs the first waveform to the controller 131. After receiving the first waveform, the controller 131 may determine the resonant frequency of the ultrasonic atomization piece 12 according to the second voltage corresponding to the first waveform.

In an embodiment, the first preprocessing sub-branch 1342 includes a third capacitor C3, a third resistor R3, and a fourth resistor R4. A first end of the third resistor R3 is connected to a first end of the ultrasonic atomization piece 12, a second end of the third resistor R3 is connected to a first end of the third capacitor C3, a first end of the fourth resistor R4, and the rectification sub-branch 1343, and a second end of the third capacitor C3 and a second end of the fourth resistor R4 are connected to the ground GND.

In this embodiment, a combination of the third resistor R3 and the fourth resistor R4 is configured to play a role of dividing a voltage, and the third capacitor C3 is configured to play a role of filtering.

In an embodiment, the rectification sub-branch 1343 includes a first diode D1. A positive electrode of the first diode D1 is connected to a connection point between the third resistor R3 and the fourth resistor R4 in the first preprocessing sub-branch 1342, and a negative electrode of the first diode D1 is connected to each of the energy storage sub-branch 1341 and the voltage limiting and current limiting sub-branch 1344.

In this embodiment, because of unilateral conductivity of the first diode D1, the first diode D1 only allows a signal greater than 0, which is equivalent to filtering out a negative half part of a first driving sub-signal output by the first preprocessing sub-branch 1342, and only remaining a positive half part. In addition, the first diode D1 can further effectively prevent a voltage of a circuit connected to the negative electrode of the first diode D1 from flowing back to a circuit connected to the positive electrode of the first diode D1, and can protect the circuit (for example, the ultrasonic atomization piece 12) connected to the positive electrode of the first diode D1.

In an embodiment, the energy storage sub-branch 1341 includes a sixteenth capacitor C16. After passing through the rectification sub-branch 1343 and the first preprocessing sub-branch 1342, a first end of the sixteenth capacitor C16 is connected to each of a second end of a first inductor L1 in a first boost branch 142, a third end of a first switch Q1 in a first switch branch 141, and the ultrasonic atomization piece 12, and a second end of the sixteenth capacitor C16 is connected to the ground GND.

Specifically, the first sub-voltage output by the rectification sub-branch 1343 can charge the sixteenth capacitor C16. When a voltage at both ends of the sixteenth capacitor C16 is a relatively stable voltage, for example, the voltage at the both ends of the sixteenth capacitor C16 is within a preset voltage range, in this case, the voltage at the both ends of the sixteenth capacitor C16 is recorded as the first detection voltage. It can be learned that, the sixteenth capacitor C16 can be driven by the first sub-voltage to perform charge accumulation to obtain a variable that can reflect an amplitude and a frequency of the first sub-voltage, and then input the variable to the controller 131. Therefore, a signal received by the controller 131 is not a rapidly changing quantity, but a variable obtained through accumulation. In other words, a processing requirement on the controller 131 is not high. Even if the controller 131 with a relatively low sampling frequency is selected, a sampling requirement on the first detection voltage can be met. Therefore, the controller 131 with the relatively low sampling frequency may be selected to reduce costs.

In an implementation, a capacitance value of the sixteenth capacitor C16 is less than or equal to 100 nF. The sixteenth capacitor C16 that is less than or equal to 100 nF is selected, so that a risk that the first capacitor C1 is damaged due to breakdown can be reduced while generating a stable first detection voltage at a relatively high speed, which is beneficial to improving work stability of the ultrasonic atomizer 100.

In an embodiment, the voltage limiting and current limiting sub-branch 1344 further includes a sixth resistor R6 and a seventh resistor R7. A first end of the sixth resistor R6 is connected to a first end of the seventh resistor R7, the rectification sub-branch 1343 is connected to the energy storage sub-branch 1341, a second end of the sixth resistor R6 is connected to the ground GND, and a second end of the seventh resistor R7 is connected to the controller 131.

In this embodiment, the sixth resistor R6 is configured to provide a relatively small load to limit the first detection voltage, so as to avoid an excessively large voltage increase of the first detection voltage. The seventh resistor R7 is configured to limit a current of the first detection voltage to prevent an excessively large current from flowing into the controller 131, so as to protect the controller 131.

In an embodiment, the control circuit 13 further includes a voltage amplification branch 135. The voltage amplification branch 135 is connected between the energy storage sub-branch 1341 and the controller 131.

Specifically, the voltage amplification branch 135 is configured to amplify a voltage obtained by energy storage of the energy storage sub-branch 1341, and output a second waveform to the controller 131. The controller 131 is further configured to determine the resonant frequency of the ultrasonic atomization piece 12 according to a third voltage corresponding to the second waveform.

In this embodiment, the voltage amplification branch 135 is arranged, which is helpful to obtain a more apparent waveform to more accurately determine the resonant frequency of the ultrasonic atomization piece 12.

In an embodiment, the voltage amplification branch 135 further includes a first resistor R1, a second resistor R2, a fourth capacitor C4, a fifth capacitor C5, and a first amplifier U1. For a connection relationship between the components, refer to FIG. 5, and details are not described herein again.

Specifically, the first amplifier U1 can amplify the voltage obtained by the energy storage of the energy storage sub-branch 1341 by a particular multiple, and the multiple is determined by performance of the first amplifier U1, so as to increase degree of distinguishing different voltage values, which is beneficial to improving precision of determining the resonant frequency of the ultrasonic atomization piece 12.

In some other embodiments, refer to FIG. 7, the control circuit 13 further includes a second conversion branch 136. The second conversion branch 136 is connected between the ultrasonic atomization piece 12 and the controller 131.

Specifically, the second conversion branch 136 is configured to convert a waveform of the first voltage into a third waveform having an interval voltage signal, and output the third waveform to the controller 131, where the voltage signal includes a rising edge or a falling edge. The controller 131 is further configured to determine the resonant frequency of the ultrasonic atomization piece 12 according to a fourth voltage corresponding to the voltage signal in the third waveform.

When no voltage signal appears, the third waveform is maintained unchanged. Moreover, the voltage signal appears only when a preset voltage position is reached each time. For example, when the preset position is a peak value of the first voltage, that is, when the first voltage reaches the peak value, the voltage signal appears in the third waveform once. Therefore, the resonant frequency of the ultrasonic atomization piece 12 can be determined according to the fourth voltage corresponding to the voltage signal. The peak value of the first voltage may be directly obtained by the controller 131 according to the waveform of the first voltage, or may be determined by a circuit structure. The following provides an embodiment of the circuit structure for obtaining the peak value.

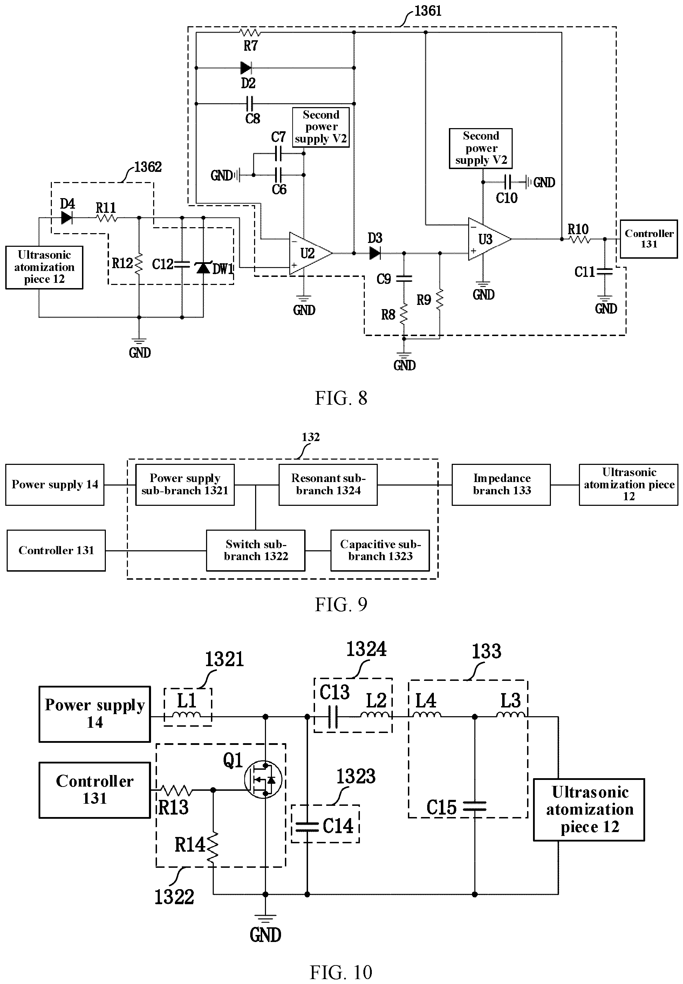

In some embodiments, as shown in FIG. 8, the second conversion branch 136 includes a peak value obtaining sub-branch 1361. The peak value obtaining sub-circuit 1361 is connected between the ultrasonic atomization piece 12 and the controller 131.

Specifically, the peak value obtaining sub-branch 1361 is configured to obtain a peak value of the waveform of the first voltage to output a third waveform. The third waveform generates the voltage signal once when the peak value is obtained each time.

Further, in an embodiment, the controller 131 is specifically configured to: output N driving frequencies to drive the ultrasonic atomization piece, where N is an integer ≥2; and obtain a third waveform under an action of M driving frequencies of the N driving frequencies to obtain M third waveforms. The fourth voltage corresponding to the third waveform is obtained. The resonant frequency of the ultrasonic atomization piece is determined according to a frequency corresponding to a minimum value of the fourth voltage.

In an actual application, if the ultrasonic atomization piece 12 operates at a resonant frequency point, and the impedance of the ultrasonic atomization piece 12 is minimum, the voltage at both ends of the ultrasonic atomization piece 12 is minimum. Because the voltage at the both ends of the ultrasonic atomization piece 12 is usually a sine wave, the peak value of the voltage at the both ends of the ultrasonic atomization piece 12 is minimum. Therefore, the resonant frequency of the ultrasonic atomization piece 12 can alternatively be determined by determining the peak value of the voltage at the both ends of the ultrasonic atomization piece 12. In addition, when the ultrasonic atomization piece 12 operates at the resonant frequency, the first voltage at both ends of the ultrasonic atomization piece 12 is a minimum voltage, and in this case, the peak value of the first voltage is the minimum. Therefore, if a detected peak value of the first voltage tends to increase as the driving frequency increases, the peak value of the first voltage does not need to be obtained at a subsequent driving frequency, that is, the third waveform does not need to be collected to improve work efficiency.

Then, each third waveform may determine one fourth voltage, and M third waveforms may determine M fourth voltages in total. A minimum value of the M fourth voltage is obtained, and a frequency corresponding to the minimum value is the resonant frequency of the ultrasonic atomization piece 12.

Referring back to FIG. 6, in an actual application, when the driving frequency is 2.92 MHz, a peak value of the first voltage is 29.18 mv; when the driving frequency is 2.94 MHz, a peak value of the first voltage is 26.67 mv; and when the driving frequency is 2.96 MHz, a peak value of the first voltage is 44.92 mv. It can be learned that, when the driving frequency is 2.94 Mhz and is exactly the resonant frequency, the peak value of the first voltage is minimum, and opposite conditions also hold true. Therefore, if a minimum value of the M fourth voltages (that is, peak values of the first voltage) at the M driving frequencies is obtained, a frequency corresponding to the minimum value is the resonant frequency of the ultrasonic atomization piece 12.

In an embodiment, the peak value obtaining sub-branch 1361 includes a sixth capacitor C6, a seventh capacitor C7, an eighth capacitor C8, a ninth capacitor C9, a tenth capacitor C10, an eleventh capacitor C11, a second diode D2, a third diode D3, a seventh resistor R7, an eighth resistor R8, a ninth resistor R9, a tenth resistor R10, a second amplifier U2, and a third amplifier U3. For a connection relationship between the components, refer to FIG. 8, and details are not described herein again.

In this embodiment, the ninth capacitor C9 is charged by the first voltage, and a voltage of the ninth capacitor C9 is charged to be equal to the peak value of the first voltage and maintained. Then, when the peak value of the first voltage is not reached, a voltage at an inverting input terminal of the third amplifier U3 is maintained less than a voltage at a non-inverting input terminal of the third amplifier U3. Only when the peak value of the first voltage is reached, the voltage at the inverting input terminal of the third amplifier U3 is maintained equal to the voltage at the non-inverting input terminal of the third amplifier U3, and a voltage output by the third amplifier U3 is the peak value of the first voltage (that is, corresponding to the voltage signal in the third waveform).

In an embodiment, the second conversion branch 136 further includes a second preprocessing sub-branch 1362. The second preprocessing sub-branch 1362 is connected between the ultrasonic atomization piece 12 and the peak value obtaining sub-branch 1361.

The second preprocessing sub-branch 1362 is configured to rectify, divide, and filter the first voltage.

In some implementations, the second preprocessing sub-branch 1362 includes a fourth diode D4, a zener diode DW1, an eleventh resistor R11, a twelfth resistor R12, and a twelfth capacitor C12. For a connection relationship between the components, refer to FIG. 8, and details are not described herein again.

In this implementation, the fourth diode D4 is configured to rectify, the eleventh resistor R11 and the twelfth resistor R12 are configured to divide a voltage, the twelfth capacitor C12 is configured to filter, and the zener diode DW1 is configured to clamp a voltage input to the non-inverting input terminal of the third amplifier U3.

Refer to FIG. 9, which is a schematic structural diagram of a control circuit 13 according to another embodiment. As shown in FIG. 9, the control circuit 13 includes a driver 131, a driving branch 132, and an impedance branch 133. The driving branch 132 includes a power supply sub-branch 1321, a switch sub-branch 1322, a capacitive sub-branch 1323, and a resonant sub-branch 1324.

The power supply sub-branch 1321 is connected to a power supply 14. The switch sub-branch 1322 is connected to each of the controller 131 and the power supply sub-branch 1321. The capacitive sub-branch 1323 is connected to the switch sub-branch 1322. The resonant sub-branch 1324 is connected to each of the power supply sub-branch 1321, the switch sub-branch 1322, and the impedance branch 133. The impedance branch 133 is further connected to the ultrasonic atomization piece 12.

Specifically, the power supply sub-branch 1321 is configured to generate direct current according to the power supply 14. The switch sub-branch 1322 is configured to be turned on or turned off in response to a first pulse signal. The capacitive sub-branch 1323 is configured to implement soft turn-on or soft turn-off of the switch sub-branch 1322. The resonant sub-branch 1324 is configured to resonate in response to switch-on and switch-off of the switch sub-branch 1322 to output a driving voltage according to the pulse voltage to drive the ultrasonic atomization piece 12. The impedance branch is configured to match a combined impedance of the impedance branch 133 and the ultrasonic atomization piece 12 with an impedance of the driving branch 132. The controller 131 is configured to obtain a voltage of the ultrasonic atomization piece 12, and determine a resonant frequency of the ultrasonic atomization piece according to the voltage of the ultrasonic atomization piece 12.

In this embodiment, when the ultrasonic atomization piece 12 needs to be driven, first, the power supply 14 is converted into a direct current for output after passing through the power supply sub-branch 1321, and meanwhile, the controller 131 outputs a first pulse signal to control the switch sub-branch 1322 to constantly cyclically switch between on and off, so as to convert the direct current output by the power supply sub-branch 1321 into an alternating current, that is, a pulse voltage. Then, after resonance occurs in the resonant sub-branch 1324, a received pulse voltage can be boosted, and the ultrasonic atomization piece 12 may be driven by using a boosted driving voltage. Because the resonant sub-branch 1324 achieves resonance, the resonant sub-branch 1324 essentially presents purely resistive, which can reduce a reactive part of the resonant sub-branch 1324, that is, reduces a power loss, thereby improving work efficiency of the ultrasonic atomizer 100. Moreover, in this case, an impedance of the resonant sub-branch 1324 is minimum, a current is maximum, a relatively large driving voltage may be output to drive the ultrasonic atomization piece 12 to stably operate.

In addition, the ultrasonic atomization piece 12 may be equivalent to a capacitive load, and after the resonance occurs in the resonant sub-branch 1324, a combination of the power supply sub-branch 1321, the switch sub-branch 1322, and the resonant sub-branch 1324 is a purely resistive output. If energy is directly transmitted between the two (that is, the capacitive load and the purely resistive output), relatively large reactive power is generated, thereby greatly reducing efficiency of driving the ultrasonic atomization piece 12.

Therefore, in this embodiment, the impedance branch 133 is further arranged, so that an impedance of a combination of the impedance branch 133 and the ultrasonic atomization piece 12 matches an impedance of a combination of the power supply sub-branch 1321, the switch sub-branch 1322, and the resonant sub-branch 1324. Therefore, a reactive power part of the combination of the impedance branch 133 and the ultrasonic atomization piece 12 may be reduced to reduce power loss. The ultrasonic atomization piece 12 can obtain relatively high driving energy, thereby improving efficiency of driving the ultrasonic atomization piece 12, and improving work efficiency of the ultrasonic atomizer 100.

Specifically, in an implementation, an impedance (Zh) of the combination of the impedance branch 133 and the ultrasonic atomization piece 12 includes a real impedance part (Rh) and an imaginary impedance part (j*Xh). When the real impedance part is equal to an impedance (Z0) of the combination of the power supply sub-branch 1321, the switch sub-branch 1322, and the resonant sub-branch 1324, and the imaginary impedance part is less than a first preset threshold, the impedance of the combination of the impedance branch 133 and the ultrasonic atomization piece 12 matches the impedance of the combination of the power supply sub-branch 1321, the switch sub-branch 1322, and the resonant sub-branch 1324. The first preset threshold may be set according to an actual application case. This is not specifically limited in this embodiment of this application.

Zh=Rh+j*Xh. Moreover, because the impedance of the combination of the power supply sub-branch 1321, the switch sub-branch 1322, and the resonant sub-branch 1324 is purely resistive, Z0=R0, where R0 represents the resistance of the combination of the switch sub-branch 1322 and the resonant sub-branch 1324. Therefore, to satisfy that the impedance of the combination of the impedance branch 133 and the ultrasonic atomization piece 12 matches the impedance of the combination of the power supply sub-branch 1321, the switch sub-branch 1322, and the resonant sub-branch 1324, conditions that need to be satisfied are: Rh=R0, and j*Xh=0. In this case, the work efficiency of the ultrasonic atomization piece 12 is relatively high.

In an embodiment, as shown in FIG. 10, the power supply sub-branch 1321 includes a first inductor L1. A first end of the first inductor L1 is connected to the power supply 14, and a second end of the first inductor L1 is connected to each of the switch sub-branch 1322 and the resonant sub-branch 1324.

Specifically, the first inductor L1 is a high-frequency choke. The high-frequency choke only has a relatively significant blocking effect on a high-frequency alternating current, has a very low blocking effect on a low-frequency alternating current, and has a lower blocking effect on a direct current. Therefore, the high-frequency choke may be configured to "enable a direct current to pass, resist an alternating current, enable a low frequency to pass, resist a high frequency". Therefore, the first inductor L1 may allow a direct current to pass through to provide energy for a subsequent circuit, that is, implement a process of outputting the direct current according to the power supply 14. In addition, the first inductor L1 may be further configured to prevent a high-frequency short circuit.

FIG. 4 further exemplarily shows a structure of a switch sub-branch 1322. As shown in FIG. 4, the switch sub-branch 1322 includes a switch tube Q1. A first end of the switch tube Q1 is connected to the controller 131, a second end of the switch tube Q1 is connected to the ground GND, and a third end of the switch tube Q1 is connected to the power supply sub-branch 1321 and the resonant sub-branch 1324.

In this embodiment, an example in which the switch tube Q1 is an N-type metal-oxide-semiconductor field-effect transistor (that is, an NMOS transistor) is taken. Specifically, a gate of the NMOS transistor is the first end of the switch tube Q1, a source of the NMOS transistor is the second end of the switch tube Q1, and a drain of the NMOS transistor is the third end of the switch tube Q1.

In addition, in another embodiment, the switch tube Q1 may alternatively be a P-type metal-oxide-semiconductor field-effect transistor or a signal relay. The switch tube Q1 may alternatively be at least one of a triode, an insulated gate bipolar transistor, an integrated gate commutated thyristor, a gate turn-off thyristor, a junction field-effect transistor, an MOS controlled thyristor, a gallium nitride-based power device, a silicon carbide-based power device, or a thyristor.

In an embodiment, the switch sub-branch 1322 further includes a thirteenth resistor R13 and a fourteenth resistor R14 connected in series. A first end of a circuit formed by connecting the thirteenth resistor R13 and the fourteenth resistor R14 in series is connected to the controller 131, a second end of the circuit formed by connecting the thirteenth resistor R13 and the fourteenth resistor R14 in series is connected to the ground GND, and a connection point between the thirteenth resistor R13 and the fourteenth resistor R14 is connected to a first end of the switch tube Q1.

In this embodiment, the thirteenth resistor R13 and the fourteenth resistor R14 are configured to divide a voltage of a first pulse signal output by the controller 131 to obtain a voltage of the first end of the switch tube Q1. When a divided voltage of the fourteenth resistor R14 is greater than a turn-on voltage of the switch tube Q1, the switch tube Q1 is turned on, or otherwise, the switch tube Q1 is turned off.

In an embodiment, the capacitive sub-branch 1323 includes a fourteenth capacitor C14, a first end of the fourteenth capacitor C14 is connected to the third end of the switch tube Q1, and a second end of the fourteenth capacitor C14 is connected to the ground GND.

Specifically, the fourteenth capacitor C14 is configured to charge when the switch tube Q1 is disconnected and a current flowing through the resonant sub-branch 1324 is less than a first current threshold, and is configured to resonate with the resonant sub-branch 1324 to discharge when the switch tube Q1 is disconnected and a current flowing through the resonant sub-branch 1324 is greater than or equal to the first current threshold. When the fourteenth capacitor C14 discharges to a second current threshold, the switch tube Q1 is turned on.

It may be understood that, a setting of the first current threshold and a setting of the second current threshold are both related to a parameter of the first capacitor C1 and a parameter of the resonant sub-branch 1324. In other words, in different application scenarios, different first current thresholds and second current thresholds may be obtained by selecting different fourteenth capacitors C14 and different resonant sub-branches 1324. This is not specifically limited in this embodiment of this application.

In this embodiment, in an instant when the switch tube Q1 is disconnected, a voltage between the second end and the third end of the switch tube Q1 does not suddenly increase, but a voltage between both ends of the first capacitor C1 is maintained first. After a current between the second end and the third end of the switch tube Q1 decreases to zero, the voltage between the second end and the third end of the switch tube Q1 starts to increase again. Therefore, soft turn-off of the switch tube Q1 is implemented.

Meanwhile, a current flowing through the resonant sub-branch 1324 is less than the first current threshold, and the fourteenth capacitor C14 is charged. Then, the current of the resonant sub-branch 1324 gradually increases until the current is greater than or equal to the first current threshold. The current of the resonant sub-branch 1324 is greater than the current of the first inductor L1, and the fourteenth capacitor C14 and the resonant sub-branch 1324 resonate to discharge. Then, when the fourteenth capacitor C14 discharges to the second current threshold, the switch tube Q1 is turned on. It can be learned that, an appropriate fourteenth capacitor C14 and an appropriate resonant sub-branch 1324 are selected, so that the second current threshold is zero, zero-voltage turned on of the switch tube Q1 may be implemented, that is, soft turn-on of the switch tube Q1 is implemented.

In this embodiment, the fourteenth capacitor C14 and the resonant sub-branch 1324 are arranged, so that a soft switching process (including soft turn-on and soft turn-off) of the switch tube Q1 can be implemented, that is, when the switch tube Q1 is turned on and turned off, a product of a voltage and a current is zero all the time. Therefore, a switching loss of the switch tube Q1 is close to zero, and switching efficiency of the switch tube Q1 is relatively high, thereby improving work efficiency of the ultrasonic atomizer 100.

FIG. 4 further exemplarily shows a structure of a resonant sub-branch 1324. As shown in FIG. 4, the resonant sub-branch 1324 includes a thirteenth capacitor C2 and a second inductor L2. A first end of the thirteenth capacitor C2 is connected to each of the power supply sub-branch 1321 (that is, the second end of the first inductor L1) and the switch sub-branch 1322 (that is, the third end of the switch tube Q1), a second end of the thirteenth capacitor C2 is connected to a first end of the second inductor L2, and a second end of the second inductor L2 is connected to the impedance branch 133.

In this embodiment, when the thirteenth capacitor C2 and the second inductor L2 form a series resonator, a circuit formed by the thirteenth capacitor C2 and the second inductor L2 is purely resistive. In this case, an impedance is minimum, and a current is maximum. A high voltage that is N times greater than a pulse voltage input to the resonant sub-branch 1324 is generated on the thirteenth capacitor C2 and the second inductor L2, where N is greater than 1. The high voltage is a driving voltage for driving the ultrasonic atomization piece 12. Therefore, the ultrasonic atomization piece 12 can obtain relatively sufficient driving energy, which is beneficial to maintaining stable operation of the ultrasonic atomization piece 12.

In an embodiment, as shown in FIG. 4, the impedance branch 133 includes a fifteenth capacitor C15, a third inductor L3, and a fourth inductor L4. A first end of the third inductor L3 is connected to a first end of the fifteenth capacitor C15 and a second end of the fourth inductor L4. A first end of the fourth inductor L4 is connected to the resonant sub-branch 1324. A second end of the third inductor L3 is connected to the ultrasonic atomization piece 12. A second end of the fifteenth capacitor C15 is connected to the ground GND.

It is to be noted that, FIG. 10 merely exemplarily shows a structure of the impedance branch 133, and in another embodiment, the impedance branch 133 may alternatively have another structure. This is not specifically limited in this embodiment of this application, as long as an impedance of a combination of the impedance branch 133 and the ultrasonic atomization piece 12 matches an impedance of the driving branch 133. For example, in an implementation, the impedance branch 133 may only include the fifteenth capacitor C15. In this case, the first end of the fifteenth capacitor C15 is connected to each of the resonant sub-branch 1324 and the ultrasonic atomization piece 12, and the second end of the fifteenth capacitor C15 is connected to the ground GND.

Meanwhile, the driving branch 132 shown in FIG. 7 may have a same structure as the driving branch 132 shown in FIG. 9. Certainly, in another embodiment, the driving branch 132 shown in FIG. 7 may alternatively have a structure such as a half-bridge circuit structure or a full-bridge circuit structure. As long as a corresponding impedance branch 133 needs to be arranged between the driving branch 132 and the ultrasonic atomization piece 12, the resonant frequency of the ultrasonic atomization piece 12 can be obtained through a solution provided in this embodiment of this application.

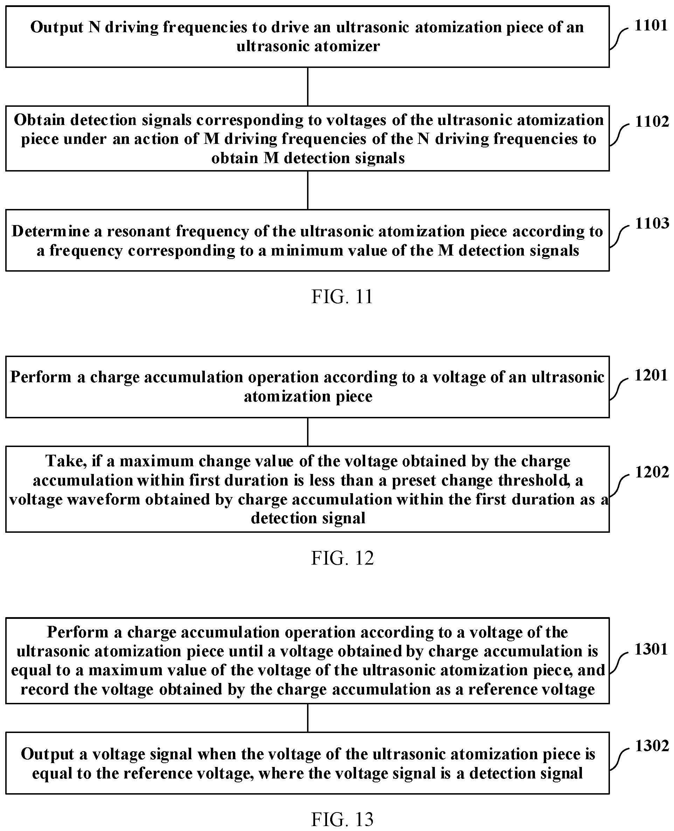

Refer to FIG. 11, which is a flowchart of a resonant-frequency determination method based on an ultrasonic atomizer according to an embodiment of this application. In some implementations, a specific structure of the ultrasonic atomizer may be implemented through structures shown in FIG. 1 to FIG. 5 and FIG. 6 to FIG. 10. A specific implementation process is described in detail in the foregoing embodiments, and details are not described herein again.

As shown in FIG. 11, the resonant-frequency determination method based on an ultrasonic atomizer includes the following steps:
Step 1101: Output N driving frequencies to drive an ultrasonic atomization piece of an ultrasonic atomizer.
Step 1102: Obtain detection signals corresponding to voltages of the ultrasonic atomization piece under an action of M driving frequencies of the N driving frequencies to obtain M detection signals.
Step 1103: Determine a resonant frequency of the ultrasonic atomization piece according to a frequency corresponding to a minimum value of the M detection signals, where
N is an integer ≥ 2, and 2≤ M ≤ N.

The detection signal is a signal related to a voltage of the ultrasonic atomization piece, for example, a peak value of the voltage of the ultrasonic atomization piece. Therefore, one detection signal may be determined at each of the M driving frequencies, and M driving frequencies may determine M detection signals in total. Because when the ultrasonic atomization piece 12 operates at the resonant frequency, the ultrasonic atomization piece 12 has a minimum impedance, in this case, the signal related to the voltage of the ultrasonic atomization piece needs to be minimum, that is, the detection signal needs to be minimum. Therefore, a minimum value of the M detection signals is obtained, and a frequency corresponding to the minimum value is the resonant frequency of the ultrasonic atomization piece 12.

In an embodiment, as shown in FIG. 12, a process of obtaining detection signals corresponding to voltages of the ultrasonic atomization piece under an action of M driving frequencies of the N driving frequencies in step 1102 includes the following steps:
Step 1201: Perform a charge accumulation operation according to a voltage of the ultrasonic atomization piece.
Step 1202: Take, if a maximum change value of the voltage obtained by the charge accumulation within first duration is less than a preset change threshold, a voltage waveform obtained by charge accumulation within the first duration as a detection signal.

Specifically, driven by the first voltage of the ultrasonic atomization piece, charge constantly accumulates. A waveform of the voltage obtained by the charge accumulation in this case is taken as the detection signal when the maximum change value (that is, a difference between a maximum value and a minimum value within the first duration) of the voltage obtained by the charge accumulation is maintained less than a preset change threshold within the first duration. In this case, it may be considered that the charge is maintained in a stable state, that is, the detection signal is a stable signal.

In another embodiment, as shown in FIG. 13, a process of obtaining detection signals corresponding to voltages of the ultrasonic atomization piece under an action of M driving frequencies of the N driving frequencies in step 1102 includes the following steps:
Step 1301: Perform a charge accumulation operation according to a voltage of the ultrasonic atomization piece until a voltage obtained by charge accumulation is equal to a maximum value of the voltage of the ultrasonic atomization piece, and record the voltage obtained by the charge accumulation as a reference voltage.
Step 1302: Output a voltage signal when the voltage of the ultrasonic atomization piece is equal to the reference voltage, where the voltage signal is a detection signal.

In this embodiment, the reference voltage is a maximum value (that is, a peak value) of the voltage of the ultrasonic atomization piece. The voltage signal is output only when the voltage of the ultrasonic atomization piece is equal to the reference voltage, the voltage signal is a maximum value of the voltage of the ultrasonic atomization piece. That is, a process of obtaining the peak value of the voltage of the ultrasonic atomization piece is implemented, the signal corresponding to the peak value is taken as the detection signal, and the resonant frequency of the ultrasonic atomization piece can be determined based on the detection signal.

It is to be understood that, for specific control and achieved beneficial effects of the ultrasonic atomizer in the method embodiment, refer to corresponding descriptions in the foregoing embodiment of the ultrasonic atomizer. For brevity, details are not described herein again.

Finally, it is to be noted that: the foregoing embodiments are merely used for describing the technical solutions of this application, but are not intended to limit this application. Under the ideas of this application, the technical features in the foregoing embodiments or different embodiments may alternatively be combined, the steps may be performed in any order, and many other changes of different aspects of this application also exists as described above, and these changes are not provided in detail for simplicity. Although this application is described in detail with reference to the foregoing embodiments, it is to be appreciated by a person skilled in the art that, modifications may still be made to the technical solutions described in the foregoing embodiments, or equivalent replacements may be made to the part of the technical features; and these modifications or replacements will not cause the essence of corresponding technical solutions to depart from the scope of the technical solutions in the embodiments of this application.

## Claims

1. An ultrasonic atomizer, **characterized by**:
a storage cavity, configured to store an atomization matrix;
an ultrasonic atomization piece, configured to generate oscillation to atomize the atomization matrix;
a control circuit and a power supply,
wherein the control circuit comprises:
a controller and a driving branch, wherein the driving branch is connected to each of the power supply and the controller, and the driving branch is configured to generate a driving voltage in response to a first pulse signal output by the controller, the driving voltage used for driving the ultrasonic atomization piece; and
an impedance branch, connected between the driving branch and the ultrasonic atomization piece, wherein the impedance branch is configured to match a combined impedance of the impedance branch and the ultrasonic atomization piece with an impedance of the driving branch, and the impedance branch comprises a first capacitor connected in parallel to the ultrasonic atomization piece,
the controller is configured to obtain a first voltage of the ultrasonic atomization piece, and determine a resonant frequency of the ultrasonic atomization piece according to the first voltage.

2. The ultrasonic atomizer according to claim 1, wherein the controller is specifically configured to:
obtain the first voltage after a first time, and determine the resonant frequency of the ultrasonic atomization piece according to the first voltage, wherein the first time is later than a time at which the ultrasonic atomization piece is started.

3. The ultrasonic atomizer according to claim 2, wherein duration between the time at which the ultrasonic atomization piece is started and the first time is any duration of [10 µs, 100 µs].

4. The ultrasonic atomizer according to claim 1, wherein the control circuit further comprises a first conversion branch;
the first conversion branch is connected between the ultrasonic atomization piece and the controller, and the first conversion branch is configured to convert a waveform of the first voltage into a first waveform fluctuating within a preset range; and
the controller is further configured to determine the resonant frequency of the ultrasonic atomization piece according to a second voltage corresponding to the first waveform.

5. The ultrasonic atomizer according to claim 4, wherein the first conversion branch comprises an energy storage sub-branch;
the energy storage sub-branch is connected between the ultrasonic atomization piece and the controller, and the energy storage sub-branch is configured to store energy in response to the first voltage; and
if a maximum change value of the voltage obtained by the energy storage of the energy storage sub-branch is less than a preset change threshold within first duration, a waveform of the voltage obtained by energy storage of the energy storage sub-branch is taken as the first waveform.

6. The ultrasonic atomizer according to claim 5, wherein the controller is specifically configured to:
output N driving frequencies to drive the ultrasonic atomization piece, where N is an integer and N≥2;
obtain first waveforms under an action of M driving frequencies of the N driving frequencies to obtain M first waveforms, where 2≤M≤N;
obtain the second voltages corresponding to the first waveforms; and
determine the resonant frequency of the ultrasonic atomization piece according to a frequency corresponding to a minimum value of the second voltages.

7. The ultrasonic atomizer according to claim 5, wherein the control circuit further comprises a voltage amplification branch;
the voltage amplification branch is connected between the energy storage sub-branch and the controller, and the voltage amplification branch is configured to: amplify a voltage obtained by the energy storage of the energy storage sub-branch, and output a second waveform to the controller; and
the controller is further configured to determine the resonant frequency of the ultrasonic atomization piece according to a third voltage corresponding to the second waveform.

8. The ultrasonic atomizer according to claim 1, wherein the control circuit further comprises a second conversion branch;
the second conversion branch is connected between the ultrasonic atomization piece and the controller, and the second conversion branch is configured to: convert a waveform of the first voltage into a third waveform having an interval voltage signal, and output the third waveform to the controller; and
the controller is further configured to determine the resonant frequency of the ultrasonic atomization piece according to a fourth voltage corresponding to the voltage signal in the third waveform.

9. The ultrasonic atomizer according to claim 8, wherein the second conversion branch comprises a peak value obtaining sub-branch; and
the peak value obtaining sub-branch is connected between the ultrasonic atomization piece and the controller, and the peak value obtaining sub-branch is configured to obtain a peak value of the waveform of the first voltage to output the third waveform, wherein the third waveform generates the voltage signal once each time the peak value is obtained.

10. The ultrasonic atomizer according to claim 8 or 9, wherein the controller is specifically configured to:
output N driving frequencies to drive the ultrasonic atomization piece, where N is an integer and N≥2;
obtain a third waveform under an action of M driving frequencies of the N driving frequencies to obtain M third waveforms, 2≤M≤N;
obtain the fourth voltages corresponding to the third waveforms; and
determine the resonant frequency of the ultrasonic atomization piece according to a frequency corresponding to a minimum value of the fourth voltages.

11. An ultrasonic atomizer, **characterized by**:
a storage cavity, configured to store an atomization matrix;
an ultrasonic atomization piece, configured to generate oscillation to atomize the atomization matrix;
a control circuit and a power supply,
wherein the control circuit comprises a controller, a driving branch, and an impedance branch;
the driving branch comprises a power supply sub-branch, a switch sub-branch, a capacitive sub-branch, and a resonant sub-branch, wherein the power supply sub-branch is connected to the power supply, the power supply sub-branch is configured to generate direct current according to the power supply, the switch sub-branch is connected to each of the controller and the power supply sub-branch, the switch sub-branch is configured to be turned on or turned off in response to a first pulse signal output by the controller to generate a pulse voltage according to the direct current, the capacitive sub-branch is connected to the switch sub-branch, the capacitive sub-branch is configured to implement soft turn-on or soft turn-off of the switch sub-branch, the resonant sub-branch is connected to each of the power supply sub-branch, the switch sub-branch and the impedance branch, and the resonant sub-branch is configured to resonate in response to turn-on and turn-off of the switch sub-branch to output a driving voltage according to the pulse voltage to drive the ultrasonic atomization piece;
the impedance branch is connected between the driving branch and the ultrasonic atomization piece, and the impedance branch is configured to match a combined impedance of the impedance branch and the ultrasonic atomization piece with an impedance of the driving branch; and
the controller is configured to obtain a voltage of the ultrasonic atomization piece, and determine a resonant frequency of the ultrasonic atomization piece according to the voltage of the ultrasonic atomization piece.

12. The method according to claim 11, wherein the controller is specifically configured to:
output N driving frequencies to drive the ultrasonic atomization piece, where N is an integer and N≥2;
obtain detection signals corresponding to voltages of the ultrasonic atomization piece under an action of M driving frequencies of the N driving frequencies to obtain M detection signals, where 2≤M≤N; and
determine the resonant frequency of the ultrasonic atomization piece according to a frequency corresponding to a minimum value of the M detection signals.

13. A resonant frequency determination method based on an ultrasonic atomizer, **characterized in that** the method comprises:
outputting N driving frequencies to drive an ultrasonic atomization piece in the ultrasonic atomizer, where N is an integer and N≥2;
obtaining detection signals corresponding to voltages of the ultrasonic atomization piece under an action of M driving frequencies of the N driving frequencies to obtain M detection signals, where 2≤M≤N; and
determining a resonant frequency of the ultrasonic atomization piece according to a frequency corresponding to a minimum value of voltages corresponding to the M detection signals.

14. The method according to claim 13, wherein said obtaining detection signals corresponding to voltages of the ultrasonic atomization piece under an action of M driving frequencies of the N driving frequencies comprises:
performing a charge accumulation operation according to a voltage of the ultrasonic atomization piece; and
if a maximum change value of the voltage obtained by the charge accumulation within first duration is less than a preset change threshold, determining a voltage waveform obtained by the charge accumulation within the first duration as a detection signal.

15. The method according to claim 13, wherein said obtaining detection signals corresponding to voltages of the ultrasonic atomization piece under an action of M driving frequencies of the N driving frequencies comprises:
performing a charge accumulation operation according to a voltage of the ultrasonic atomization piece until a voltage obtained by the charge accumulation is equal to a maximum value of the voltage of the ultrasonic atomization piece, and recording the voltage obtained by the charge accumulation as a reference voltage; and
outputting a voltage signal when the voltage of the ultrasonic atomization piece is equal to the reference voltage, wherein the voltage signal is a detection signal.
